# EUROPEAN PATENT APPLICATION

(11) **EP 1 223 166 A1**
(43) Date of publication of application: **17.07.2002**
(21) Application number: 02001112.8
(22) Date of filing: 16.12.1992
(51) Int. Cl.: C07D 257/02, C07D 401/14, C07D 409/14, C07D 471/04, A61K 31/395, A61K 31/4427, A61P 31/18

(54) **Linked cyclic polyamines with activity against hiv.**

(30) Priority: 16.12.1991 GB 9126677
(62) Divisional of application: 93900285.3
(71) Applicant: AnorMED Inc., Langley BC V2Y 1N5 (CA)
(72) Inventor: The designation of the inventor has not yet been filed
(74) Representative: W.P. Thompson & Co.

(57) **Abstract**

There is disclosed a pharmaceutical composition comprising as active ingredient a linked cyclic compound of general formula I,

Z - R - A - R' - Y (I)

in which Z and Y are independently cyclic polyamine moieties having from 9 to 32 ring members and from 3 to 8 amine nitrogens in the ring spaced by 2 or more carbon atoms from each other,
A is an aromatic or heteroaromatic moiety,
R and R' are each a substituted or unsubstituted alkylene chain or heteroatom containing chain.
or an acid addition salt or metal complex thereof, in admixture or association with a pharmaceutically acceptable diluent or carrier.

## Description

This invention concerns improvements in chemical compounds, more especially it concerns compounds and pharmaceutical compositions. In particular it concerns compositions and compounds having activity in in vitro tests on Human Immunodeficiency Virus-infected cells.

The disease known as Acquired Immune Deficiency Syndrome (AIDS) caused by infection by HIV has attracted immense research effort because of the effects of the disease on infected individuals and the dangers of the disease spreading to a wider section of the population. In general, although various chemo-therapeutic treatments have been advocated, and some compounds have emerged as a potential basis for treatment, there is still a need for alternatives. In particular, most treatments such as the compound known as AZT have a high toxicity to cells, and it would be desirable to find compounds which are less toxic. In man, the development of resistance to AZT has been identified as an additional clinical problem.

We have found a group of compounds which show protective properties in in vitro screens of cells challenged with HIV-1 and/or HIV-2, and are therefore useful for the treatment of AIDS and AIDS Related Complex and other viral and especially retroviral infections. Accordingly, the present invention provides the use of compounds defined below, in pharmaceutical compositions for treating HIV-infected patients. The invention further provides pharmaceutical compositions comprising a said compound in combination or association with a pharmaceutically acceptable diluent or excipient, for the treatment of HIV-infected patients. The invention may also be defined as the use of a said compound for the manufacture of a medicament for the treatment of HIV-infected patients. The invention further provides a process for the production of a pharmaceutical composition for the treatment of a HIV-infected patient, comprising the combination of a compound as defined below with a pharmaceutically acceptable diluent or excipient, and formulating said composition into a form suitable for administration to said patient. The invention also provides a method of treatment of an HIV-infected patient, comprising administering to said patient an effective dose of a said compound. It is to be understood that treatment includes prophylactic treatment of patients at risk, in view of the protective properties observed. The use of the compounds may also be stated as a method of treating HIV-infected or HIV-challenged human cells to prevent or modulate the multiplication of the HIV, comprising administering to said cells an effective dose of a said compound. Whilst this description is especially directed to combating HIV, this invention includes other aspects in which other diseases may be treated, including for example microbial infections.

A 2,2'-dimer of cyclam has been reported as being isolated as a 2% by-product in the synthesis of cyclam (1,4,8,11-tetraazacyclotetradecane) (Barefield et al, JCS Chem Comm (1981), 302). This compound was stated to be insoluble in water. We believe that the insoluble 2,2'-bicyclam is a mixture of the 2R,2'R and 2S,2'S enantiomers; we have characterised a soluble dimer which we believe to be the meso 2R,2'S isomer. The 6,6'-bicyclam isomer has been reported by Fabbrizzi et al, Inorg Chem 25, 2671 (1986). Certain N,N'-linked bicyclic compounds have been reported by Ciampolini et al, Inorg Chem 26, 3527 (1987). No biological activity has been suggested for such compounds.

US Patent 4,156,683 discloses monocyclic and bicyclic macrocyclic compounds, which are said to have biological activity in regulating sodium, potassium and calcium levels in mammals. Additionally, a specific group of N-alkylated monocyclic compounds are said to possess activity against A₂ influenza viruses in a modified Hermann test on chick fibroblast tissue. It is also said that the preferred compounds, which form complexes of greater stability, are those having three bridging chains between bridgehead nitrogen atoms, that is fused bicyclic compounds.

Our USP 5,021,409 describes linked cyclic compounds as being active against HIV-1 and HIV-2 in in vitro tests. We have now discovered that certain of these linked cyclic compounds exhibit surprisingly improved activity against HIV. Thus, the present invention concerns a selected group of the compounds taught in said USP, having activity of at least an order of magnitude greater than the compounds tested in said USP.

The present invention provides as active compounds linked cyclic compounds of the general formula I

Z - R - A - R' - Y (I)

in which Z and Y are independently cyclic polyamine moieties having from 9 to 32 ring members and from 3 to 8 amine nitrogens in the ring spaced by 2 or more carbon atoms from each other,
A is an aromatic or heteroaromatic moiety, and
R and R' are each a substituted or unsubstituted alkylene chain or heteroatom-containing chain which spaces the cyclic polyamines and the moiety A. The invention also encompasses acid addition salts and metal complexes of the compounds of formula I.

In the above formula, the cyclic polyamine moieties may be substituted or unsubstituted, and suitable substituents are alkyl and/or aryl groups, eg of up to 10 carbon atoms, and any other atoms or groups which do not substantially adversely affect the activity or toxicity of the compounds. Preferred moieties are those of 9 to 30 ring members, especially 9 to 20 ring members, most preferably 10 to 15 ring members, and preferred numbers of amine nitrogen atoms are 3 to 6. The moieties may be linked by attachment to the carbon atoms or nitrogen atoms of the polyamine ring, ie C-C', C-N', N-N'; preferably, the moieties are linked at nitrogen atoms. Y and Z may be identical or non-identical, although it is convenient that they are identical.

The aromatic or heteroaromatic moiety A tethers Y and Z through the linking groups R and R'. Moiety A may be phenyl or fused aromatic such as napthyl, heterocyclic such as pyridyl or thiophenyl, fused heterocyclic or joined aromatic and/or joined heteroaromatic, for example biphenyl or bipyridyl respectively. The moieties A may also be substituted at single or multiple non-linking positions with electron-donating groups, eg alkyl, thio, thioalkyl, hydroxyl, alkoxyl, amino and derivatives thereof, or electron-withdrawing groups or atoms, eg nitro, halogen, carboxy, arboxamido, sulfonic add and derivatives thereof.

The linking group R may contain heteroatoms, eg O, N or S, and may be saturated, unsaturated or polyunsaturated, and is preferably alkyl or cycloalkyl of 1 to 12 carbon atoms, more preferably alkyl of 1 to 6 carbon atoms, especially alkyl of 1 to 3 carbon atoms.

The invention also includes what may be termed "pro-drugs", that is protected forms of the linked cyclic compounds, which release the compound after administration to a patient. For example, the compound may carry a protective group which is split off by hydrolysis in body fluids, eg in the bloodstream, thus releasing active compound. A discussion of pro-drugs may be found in "Smith and Williams' Introduction to the Principles of Drug Design", H J Smith, Wright, 2nd Edition, London 1988.

A few of the active compounds according to the invention are known, (see Inorg Chem 26 (1987), p 3527-3533 and J Chem Soc, Chem Commun, (1991), 206, 207).

Accordingly, certain of the compounds of formula I are novel. The invention accordingly provides novel linked cyclic polyamine compounds of general formula Ia,

Z - R - A' - R' - Y (Ia)

in which Z, Y, R and R' are as defined above, with R and R' linked to nitrogen atoms in Z and Y, and
A' is an aromatic or heteroaromatic moiety which is unsubstituted or substituted, provided that A' is not unsubstituted phenylene when Z and Y are 14-membered tetraaza rings, and R and R' are both methylene,
and their acid addition salts and metal complexes.

The invention further provides a method for the production of the compounds of formula Ia, which method comprises nucleophilic attack by cyclic polyamines Z' and Y' each having a single unprotected ring amine nitrogen, all other ring amine nitrogens being protected, on a compound of formula II

X - R - A' - R' - X II

wherein R, R' and A' are as defined above, and
each X is an active substituent which can be displaced by the unprotected amine nitrogens of polyamines Z' and Y' and is preferably selected from Br, Cl, I, methanesulfonate, 4-tolylsulfonate and trifluoromethane sulfonate, and subsequently deprotecting the ring amine nitrogens.

It is well within the capabilities and knowledge of the skilled synthetic chemist to protect the amine nitrogens of cyclic polyamines, and it is preferred to use substitution by methanesulfonyl and/or 4-tolylsulfonyl and/or diethylphosphoryl. The compounds of formula II are known.

The reaction is preferably carried out in a solvent, such as acetonitrile or dimethylformamide, tetrahydrofuran or dioxane and in the presence of a base, for example sodium carbonate or potassium carbonate. The reaction generally takes place readily at room temperature to elevated temperature, to give a linked molecule having protected amine nitrogen atoms. In general, a mixture of products will be obtained, and we have found that chromatography on silica gel is a particularly convenient method of separation.

The deprotection step is suitably carried out by refluxing the protected molecule in a mixture of aqueous HBr and acetic acid or in the case of diethylphosphoryl in the presence of hydrogen chloride (gas) in THF or dioxane.

It is convenient that Z and Y are identical, so that the compound of formula II is reacted with two equivalents of the protected polyamine.

In the case where Z and Y are not identical, it is appropriate to modify the process described above, using as reactant a compound of formula IIa, 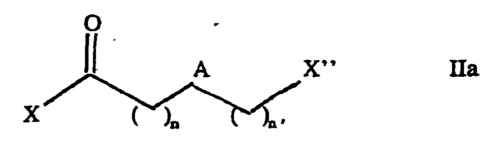 in which A is as defined above,
X' is a halogen, preferably bromine or chlorine
X" is a halogen, preferably bromine or iodine, or tosyl or mesyl, and
n = 0 or a positive integer such as to yield chain R in the product, and
n' = a positive integer such as to yield chain R' in the product,
and reacting firstly with a protected cyclic polyamine Z' and secondly reacting the reaction product with a protected cyclic polyamine Y', subsequently reducing the carbonyl group on the chain, and thereafter deprotecting the ring amine nitrogens.

The first stage reaction is conveniently carried out in a solvent, for example dichlorometbane or chloroform with triethylamine, and the second stage reaction is conveniently carried out under the conditions described above, that is in a solvent and in the presence of a base. Before deprotection, which may be accomplished as described above, it is necessary to reduce the carbonyl group on the linking chain using a reducing agent such as borane or lithium aluminium hydride, in manner generally known. The skilled synthetic chemist will be able to carry into effect the process of the invention in its various stages and possible variants.

The compounds are indicated for the treatment of viral infections, especially retrovirus infections and particularly HIV infections, and the compounds of formula I are to be considered as active compounds for the pharmaceutical compositions, processes for making the same and methods of treatment mentioned above. In these aspects of the invention, it is to be understood that meso forms, enantiomers and resolved optically active forms of the compounds of formula I are included. Also, it is to be considered within the invention, compounds of formula I diluted with non-toxic or other active substances.

Add addition salts, for example hydrochlorides, and non-toxic labile metal complexes of the compounds of formula I are also active compounds according to the present invention. Non-toxic in the present context has to be considered with reference to the prognosis for the infected patient without treatment. Copper and zinc complexes are preferred although other metals such as nickel may be considered, whereas less labile metal atoms such as cobalt and rhodium are less preferred because of likely lower selectivity.

The present invention will now be illustrated by the following preparative examples.

### EXAMPLE 1

### a) 2,3,5,6-Tetrafluoro-p-xylene-∝,∝'-diol

To a stirred solution of perfluoroterephthalic acid (1.0g, 4.2mmol) in anhydrous THF (10ml) under an atmosphere of dry argon was added Borane. THF complex (1.0M solution in THF, 10 equivalents, 42ml) dropwise, and the mixture stirred at room temperature overnight. The solution was evaporated under reduced pressure to give a colourless oil and the excess Borane destroyed by addition of anhydrous methanol (40ml) and evaporation (repeated three times). The residue was treated with 5% aqueous hydrochloric acid then the pH of the mixture was adjusted to pH9 with 1N aqueous sodium hydroxide solution and extracted with dichloromethane (3 x 50ml). The combined organic extracts were dried (MgSO₄) and evaporated to give 2,3,5,6-tetra-fluoro-p-xylene-∝,∝'-diol (0.75 g, 86%) as a white solid. This was used without further purification.

### b) 2,3,5,6-Tetrafluoro-p-xylene-∝,∝'-diol dimesylate

To a stirred solution of 2,3,5,6-tetrafluoro-p-xylene- α,α'-diol (0.72g, 3.4mmol) in dichloromethane (40ml) containing triethylamine (1.2ml, 2.5 equivalents) was added methanesulfonyl chloride (0.58ml, 2.2 equivalents) dropwise at 0°C and the mixture was allowed to warm to room temperature overnight. The solution was washed with saturated aqueous sodium bicarbonate solution (2 x 20ml) and brine (2x 20ml) then dried (MgSO₄) and evaporated under reduced pressure. The residue was suspended in ether and filtered giving 2,3,5,6-tetrafluoro-p-xylene-∝,∝'-diol dimesylate (0.9g, 72%) as a white solid.

### c) 1,1'-[2,3,5,6-Tetrafluoro-1,4-phenylenebis-(methylene)]-bis-tris(p-toluenesulfonyl)-1,4,8,11-tetraazacyclotetradecane

2,3,5,6-Tetrafluoro-p-xylene-∝,∝'-diol dimesylate (150mg, 0.4mmol), tris-(p-toluenesulfonyl)-1,4,8,11-tetraazacyclotetradecane monohydrate 826mg, 1.2mmol, 3.0 equivalents) and potassium carbonate (252mg, 3.0 equivalents) in anhydrous acetonitrile (20ml) were heated to reflux with stirring under argon for 48 hours until all the dimesylate starting material had been consumed; confirmed by TLC (silica gel, 2% methanol in dichloromethane as eluent). The mixture was evaporated under reduced pressure and the residue was dissolved in ethyl acetate (40ml) and washed with saturated aqueous sodium bicarbonate solution (2 x 20ml) and brine (2 x 20ml) then dried (MgSO₄) and evaporated. The residue was purified by column chromatography on silica gel eluting with 2% methanol in dichloromethane giving a white foam identified by ¹H NMR and FAB-MS as 1,1'-[2,3,5,6-tetrafluoro-1,4-phenylene-bis-(methylene)]-bis-tris(p-toluenesulfonyl)-1,4,8,11-tetraazacyclotetradecane. C₇₀H₈₆N₈O₁₂S₆F₄ requires C, 56.05; H, 5.78; N, 7.47; found C, 55.81; H, 5.73; N, 7.36.

### d) 1,1'-[2,3,5,6-Tetrafluoro-1,4-phenylenebis-(methylene)]-bis-1,4,8,11-tetraazacyclotetradecane

1,1'-[2,3,5,6-Tetrafluoro-1,4-phenylenebis-(methylene)]-bis-tris-(p-toluenesulfonyl)-1,4,8,11-tetraazacyclotetradecane (200mg, 0.13mmol) was dissolved in a mixture of acetic acid and hydrobromic acid (48%) in a ratio of approximately 3:2 by volume (10ml) and heated to 100°C for 24 hours during which time a white solid precipitated. The mixture was allowed to cool and the solid was filtered off and washed with acetic acid and ether and dried in vacuo giving a white solid identified by ¹H NMR, FAB-MS and elemental analysis as 1,1'-[2,-3,5,6-tetra-fluoro-1,4-phenylene-bis-(methylene)]-bis-1,4,8,11-tetraazacyclotetradecane octahydrobromide dihydrate (65 mg, 40%).
C₂₈H₆₂N₈O₂Br₈F₄ requires C, 26.73; H, 4.96; N, 8.90; found C, 26.84; H, 5.05; N, 8.21.

The following compounds were prepared using analogous methods to those described above in steps b)-d):

5-Nitro-m-xylene-∝,∝'-diol gave 1,1-[5-Nitro-1,3- phenylene-bis-(methylene)]-bis-1,4,8,11-tetraazacyclotetradecane octahydrobromide dihydrate.
C₂₈H₆₅N₉O₄Br₈ requires C, 27.31; H, 5.31; N, 10.24; found C, 27.49; H, 5.26; N, 9.75.

2,4,5,6-Tetrachloro-m-xylene-∝,∝'-diol gave 1,1'-[2,4,5,6-tetrachloro-1,3-phenylene-bis-(methylene)]-bis-1,4,8,11-tetraaza-cyclotetradecane octahydrobromide dihydrate.
C₂₈H₆₂N₈O₂Cl₄Br₈ requires C, 25.40; H, 4.71; N, 8.46; found C, 25.72; H, 4.76; N, 8.05.

### EXAMPLE 2

### a) ∝,∝'-Dibromo-1,4-dimethylnaphthalene

To a solution of 1,4-dimethylnaphthalene (0.5g, 3.2mmol) and benzoyl peroxide (0.08 equivalents, 62mg) in carbon tetrachloride (20ml) was added N-bromosuccinimide (1.14g, 2.0 equivalents) and the mixture was heated to reflux for 24 hours during which time a white solid precipitated. The mixture was filtered hot (to remove the succinimide by-product) and then allowed to cool over several hours during which time a white cystalline solid precipitated. The solid was filtered off and dried giving 1,4-dimethylnaphthalene-∝,∝'- dibromide (473mg, 50%).

The following compound was prepared using methods analogous to steps c) and d) of Example 1:

1,4-Dimethylnaphthalene-∝,∝'-dibromide gave 1,1'-[1,4-naphthylenebis-(methylene)]-bis-1,4,8,11-tetraazacyclotetradecane octahydrobromide tetrahydrate.
C₃₂H₇₂N₈O₄Br₈ requires C, 30.20; H, 5.69; N, 8.81; found C, 30.28; H, 5.52; N, 8.66.

### EXAMPLE 3

### a) 1-Benzyl-5,13-di-(p-toluenesulfonyl)-9-methanesulfonyl-1,5,9,13-tetraazacyclohexadecane.

To a solution of N,N-bis-[3-(p-toluenesulfonylamidopropyl)]-benzylamine hydrochloride (25g) (NL Patent 6603655) in dry DMF (800ml) under argon was added sodium hydride (10 equivalents) in small portions over 3 hours. When the addition was complete the solution was heated at 60°C for 1 hour then allowed to cool and the excess sodium hydride was removed by filtration under argon. The filtrate was transferred to another dry flask and the solution was then heated to 100-110°C and bis-propanolamine- trimethanesulfonate [P Moore, J Chem Soc Dalton Trans 1985 (7) 1361-1364] (1.0 equivalent) in DMF (500ml) was added dropwise over 8 hours with rapid stirring. The temperature was maintained at 100-110°C for a further 16 hours, allowed to cool, then the mixture was poured into iced water (1500ml) and the resulting off-white precipitate that formed was collected by filtration. The solid was dissolved in dichloromethane (250ml) and the solution was washed with water (5 x 50ml), then dried (MgSO₄) and evaporated under reduced pressure to give a yellow oil. Trituration with ethanol (200ml) gave a white crystalline solid which was filtered off, washed with a small volume of ethanol, then ether and dried in vacuo to give 1-benzyl-5,-13-di-(p-toluenesulphonyl)-9-methanesulphonyl-1,5,9,-13tetraazacyclo-hexadecane (45%), identified by ¹H NMR and FAB-MS.

### b) 1,9-Di-(p-toluenesulfonyl)-5-methanesulfonyl-1,5,9,13-tetraazacyclohexadecane

To a solution of 1-benzyl-5,13-di-(p-toluene-sulfonyl)-9-methanesulfonyl-1,5,9,13-tetraazacyclohexadecane in formic acid (20ml) was added Palladium hydroxide on carbon (Pearlmans catalyst, 4.0g) and the resulting suspension was heated to reflux for 72 hours with stirring. The mixture was allowed to cool, then filtered through celite and the filtrate was evaporated under reduced pressure. The colourless oil which remained was dissolved in dichloromethane (50ml) and washed with 10% aqueous sodium hydroxide solution (2 x 20ml), and water (2 x 20ml) then dried (MgSO₄) and evaporated under reduced pressure. The residue was purified by column chromatography on silica gel eluting with 3 % methanol in dichloromethane giving a white solid identified by ¹H NMR and FAB-MS as 1,9-di-(p-toluene-sulphonyl-5-methanesulfonyl-1,5,9,13-tetraazacyclohexadecane.

The mono-deprotected tetraazacyclohexadecane macrocycle described in step b) was used as described in Example 1 steps c) and d), to prepare tetraazacyclohexadecane dimers.

The following compounds were prepared in this manner.

∝,∝'-Dibromo-m-xylene gave 1,1'-[1,3-phenylene-bis-(methylene)]-bis-1,5,9,13-tetraazacyclohexadecane octahydrobromide hexahydrate.
C₃₂H₇₆N₆O₆Br₈ requires C, 29.29; H, 6.15; N; 8.54; found C, 29.37; H, 5.50; N, 7.90.

∝,∝'-Dibromo-p-xylene gave 1,1'-[1,4-phenylene-bis-(methylene)]-bis-1,5,9,13-tetraazacyclohexadecane octahydrobromide hexahydrate.
C₃₂H₇₆N₈O₆Br₈ requires C, 29.29; H, 6.15; N, 8.54; found. C, 28.96; H, 5,47; N,7.96.

Other compounds which may be made according to the invention are:
1,1'-[1,3-phenylenebis(methylene)]-bis-1,5,9,13-tetraazacyclohexadecane
1,1'-[1,3-phenylenebis(methylene)]-bis-1,5,9-triazacyclododecane
1,1'-[1,4-phenylenebis(methylene)]-bis-1,5,9-triazacyclododecane

### EXAMPLE 4

### Synthesis of Compound F

### 1,1'-[1,4-Phenylene-bis-(methylene)]-bis-1,4,8,11-tetraazacyclotetradecane zinc dichloride monohydrate

To a stirred solution of 1,1'-[1,4-phenylene-bis-(methylene)]-bis-1,4,8,11-tetraazacyclotetradecane (1g) in methanol (25ml) was added zinc(II) chloride (0.54g, 2.0 eq) in methanol (5ml). Towards the end of the addition a white precipitate formed. Sufficient methanol and. water were added to give a homogenous solution and the mixture was then evaporated in vacuo. The solid residue was suspended in a mixture of methanol/ether and filtered giving 1,1'-[1,4-phenylene-bis-(methylene)]-bis-1,4,8,11-tetraazacyclotetradecane zinc dichloride monohydrate (1.45g, 94%) as a white powder.
C₂₈H₅₆N₈Cl₄O Zn₂ requires: C, 42.38; H, 7.11; N, 14,12; Cl, 17.88; found; C, 42.64; H, 7.14; N, 14.18; Cl, 17.89.

### EXAMPLE 5

### Synthesis of Compound G

### 1,1'-[1,4-Phenylene-bis-(methylene)]-bis-1,4,8,11-tetraazacyclotetradecane copper diacetate heptahydrate

To a stirred solution of 1,1'[1,4-phenylene-bis-(methylene)]-bis-1,4,8,11-tetraazacyclotetradecane (100mg) was added copper(II) acetate (72mg, 2.0 eq) in one portion. The solution became dark blue/purple in colour almost immediately. The mixture was stirred for one hour then triturated with ether to give a blue precipitate. The blue solid was filtered off and dried giving 1,1'-[1,4-phenylene-bis-(methylene)]-bis-1,4,8,11-tetraazacyclotetradecane copper diacetate heptahydrate (80mg, 46%).
C₃₆H₈₀N₈O₁₅Cu₂ requires; C, 43.58; H, 8.13; N, 11.29; found: C, 43.24; H, 7.88; N, 11.13.

### EXAMPLE 6

### z-Bis-ethyl[1,4-phenylene]-bis-(3-propenoate)

To a stirred solution of terephthalaldehyde (10.96g) in dichloromethane (50ml) was added (carbethoxymethylene)triphenylphosphorane (5.0g, 2.0 eq) in one portion and the mixture was continued stirring for 48 hours at room temperature. The solution was evaporated to dryness in vacuo and the residue was suspended in ether. The mixture was heated to boiling then cooled in an ice bath and the precipitate which formed was filtered off. The filtrate was evaporated and the solid residue was purified by column chromatography on a short plug of silica gel using dichloromethane as eluent giving a white solid, identified by ¹H NMR as z-bis-ethyl[1,4-phenylene]-bis-(3-propenoate) (1.96g, 100%).

### Bis-ethyl[1,4-phenylene]-bis-(3-propanoate)

The bis-olefin described above (1.0g) was dissolved in methanol (with heating) containing palladium on carbon (10% Pd/C, Aldrich). The mixture was hydrogenated at 50 psi for 24 hours with vigorous shaking on a Parr apparatus. The palladium on carbon was removed by filtration through celite and the solvent evaporated in vacuo giving the title compound as a colourless solid (0.98g, 100%). This was used without further purification.

### (1,4-Phenylene)-bis-(3-hydroxypropane)

To a stirred solution of the bis-ester from above (0.98g) in THF (20ml) was added borane/tetrahydrofuran (1.0M, Aldrich, 35.5ml, 10 eq) dropwise with stirring. The solution was heated to reflux for 5 hours then evaporated to dryness in vacuo. Anhydrous methanol (20ml) was added and the solution was evaporated to dryness once again. This procedure was repeated three times. The residue was suspended in 5% hydrochloric add (20ml) and then made basic with 10N sodium hydroxide solution until the pH was around pH 8-9 and extracted with ethyl acetate (3 x 50ml). The combined extracts were washed with brine (50ml) then dried (MgSO₄) and evaporated in vacuo giving a colourless solid identified by ¹H NMR as (1,4-phenylene)-bis-(3-hydroxypropane) (650mg, 95%).
This was used without further purification.

### (1,4-Phenylene)-bis-[3-(trifluoromethanesulphonyl)-propane]

To a solution of the diol from above (100mg) in CH₂Cl₂ (10ml) containing triethylamine (160µl, 2.2 eq) cooled to -78°C under argon was added trifluoromethanesulphonic anhydride (182µl, 2.1 eq) dropwise with stirring. The mixture was stirred for a further 30 minutes at -78°C then allowed to warm to room temperature. The solution was diluted with dichloromethane, washed with saturated aqueous sodium bicarbonate solution (30ml), dried (MgSO₄) and evaporated in vacuo giving (1,4-phenylene)-bis-[3-trifluoromethanesulphonyl)-propane] as a colourless oil (236g, 100%).

### 1,1'-[1,4-Phenylene-bis-(propylene))-bis-tris-(p-toluenesulphonyl)-1,4,8,11-tetraazacyclotetradecane

To a stirred solution of the bis-triflate from above (236g) in acetonitrile (10ml) was added tris-(p-toluenesulphonyl)-1,4,8,11-tetraazacyclotetradecane (746g, 2.1 eq) and potassium carbonate (216g, 3.0 eq) and the mixture was heated to reflux overnight under argon. The mixture was diluted with ethyl acetate (50ml) and washed with saturated aqueous sodium bicarbonate solution (2 x 30ml), dried (MgSO₄) and evaporated in vacuo giving a yellow foamy solid. This mixture was purified by column chromatography on silica gel using 2% methanol in dichloromethane as eluent giving a white foamy solid, identified by ¹H NMR as 1,1'[1,4-phenylene-bis-(propylene)]-bis-tris-(p-toluenesulphonyl)-1,4,8,11-tetraazacyclotetradecane (650mg, 83%).
Mass spectrum (FAB); M/e (relative intensity); 1455 (M+1, 95), 1323 (100).

### Synthesis of Compound H

### 1,1'-[1,4-Phenylene-bis-(propylene)]-bis-1,4,8,11-tetraazacyclotetradecane octahydrobromide dihydrate

The per-tosyl compound from above (275g) was dissolved in acetic acid (10ml) and aqueous hydrobromic acid (48%, Aldrich, 6ml) and the mixture was heated to reflux overnight during which time a white precipitate formed from a brown solution. The reaction was allowed to cool to room temperature and the solid was filtered off, washed with acetic acid then ether and dried in vacuo giving a white solid identified by ¹H NMR and elemental analysis as 1,1'[1,4-phenylene-bis-(propylene)-bis-1,4,8,11-tetraazacyclotetradecane octahydrobromide dihydrate (150mg, 68%).
C₃₂H₇₆N₈Br₈O₂requires; C, 30.97; H, 6.17; N, 9.03; found; C, 30.79; H, 5.96; N, 8.92. Mass spectrum (FAB); m/e (relative intensity); 641 (M+HBr, 70), 639 (M+HBr, 75), 560 (M+1, 100).

### EXAMPLE 7

### 1-[(1-Methylene-4-bromomethylene)-phenylene]-tris-(p-toluenesulphonyl)-1,4,8,11-tetraazacyclotetradecane

A solution of tris-(p-toluenesulphonyl)-1,4,8,11-tetraazacyclotetradecane (200mg) in acetonitrile (5ml) was added dropwise over I hour to a solution of α,α'-dibromo-p-xylene (0.78g, 10 eq) in refluxing acetonitrile (10ml) containing potassium carbonate (81mg, 2.0 eq). The progress of the reaction was monitored by thin layer chromatography until the starting aza-macrocycle was consumed (30 minutes approximately). The solution was diluted with dichloromethane, washed with saturated aqueous sodium bicarbonate solution (2 x 20ml), dried (MgSO₄) and evaporated in vacuo. The foamy white residue was purified by column chromatography on silica gel using 50/50 ethyl acetate/hexane as eluent giving a white solid, identified by ¹H NMR as 1-[(1-methylene-4-bromoethylene)phenylene]-tris-(p-toluenesulphonyl)-1,4,8,11-tetraazacyclotetradecane (115mg, 50%).

### 1,1'-[Phenylene-bis-(methylene)]-tris-(p-toluenesulphonyl)-1,4,8,11-tetraazacyclotetradecane)-9-methanesulphonyl-5,13-bis-(p-toluenesulphonyl)-1,5,9,13-tetraazacyclohexadecane

To a stirred solution of the bromomethyl compound described above (115mg) in acetonitrile (5ml) was added 9-methanesulphonyl-5,13-bis-(p-toluenesulphonyl)-1,5,9,13-tetraazacyclohexadecane (83mg, 1.0 eq) and potassium carbonate (38mg, 2.0 eq) and the mixture was heated to reflux overnight with stirring. The mixture was allowed to cool and diluted with dichloromethane, washed with saturated aqueous sodium bicarbonate solution, dried (MgSO₄) and evaporated. The residue was purified by column chromatography on silica gel using 5% methanol/dichloromethane as eluent giving a foamy white solid, identified by ¹H NMR as 1,1'-[1,4-phenylene-bis-(methylene)[-tris-(p-toluenesulphonyl)-1,4,8,11-tetraazacyclotetradecane)-9-methanesulphonyl-5,13-bis-(p-toluenesulphonyl)-1,5,9,13-tetraazacyclohexadecane (155mg, 82%).
Mass spectrum (FAB); m/e (relative intensity); 1379 (M+1, 100), 1223 (40).

### Synthesis of Compound I

### 1,1'-[1,4-Phenylene-bis-(methylene)]-1,4,8,11-tetraazacyclotetradecane-1,5,9,13-tetraazacyclohexadecane octahydrobromide

The per-tosylated derivative from above (155mg) was dissolved in a mixture of acetic acid (5ml) and hydrobromic acid (48%, 3ml, Aldrich) and the mixture was heated to reflux overnight with stirring during which time a white solid had precipitated from a dark brown solution. The reaction was allowed to cool and the solid filtered off, washed with ether and dried in vacuo giving a white amorphous solid identified by ¹H NMR and FAB-MS as 1,1'-[1,4-phenylene-bis-(methylene)]-1,4,8,11-tetraazacyclotetradecane-1,5,9,13-tetraazacyclohexadecane octahydrobromide (85mg).
Mass spectrum (FAB); m/e (relative intensity); 613 (M+HBr, 15), 611 (M+HBr, 15) 531 (M+1, 25), 201 (100).

### EXAMPLE 8

### 1,1'-[3,3'-Biphenylene-bis-(methylene)]-bis-tris-(p-toluenesulphonyl)-1,4,8,11-tetraazacyclotetradecane

A mixture of 3,3'-bis-(bromomethyl)-1,1'-biphenyl [W Wenner, J. Org. Chem. (1952), 17, 525-528], (200mg, 0.59mmol), anhydrous potassium carbonate (325mg, 2.35mmol, 4 eq) and tris-(p-toluenesulphonyl)-1,4,8,11-tetraazacyclotetradecane (801mg, 1.18mmol, 2 eq) in anhydrous acetonitrile (15ml) was stirred at 50°C under argon. After 6 hours the reaction mixture was allowed to cool; dichloromethane (75ml) was added and the resulting solution filtered. The filtrate was evaporated in vacuo to yield a glassy white solid. Chromatography of the crude product on a column of silica gel (2.5cm x 20cm), eluting with methanol/dichloromethane 1:160 v/v gave a white solid, identified by ¹H NMR as 1,1'-[3,3'-biphenylene-bis-(methylene)]-bis-tris-(p-toluene-sulphonyl)-1,4,8,11-tetraazacyclotetradecane (665mg, 76%).

### Synthesis of Compound J

### 1,1'-[3,3'-Biphenylene-bis-(methylene)]-bis-1,4,8,11-tetraazacyclotetradecane octahydrobromide tetrahydrate

The per-tosylated derivative from above (450mg, 0.30mmol) was dissolved in glacial acetic acid (9ml). Hydrobromic acid (∼48% w/v, Aldrich, 3.5ml) was added and the resulting mixture heated to reflux. After 24 hours the dark brown solution was cooled in an ice bath over 2 hours during which time an off-white precipitate formed. The precipitate was collected by centrifugation and washed with glacial acetic acid (3 x 10ml) followed by diethyl ether (4 x 10ml) then dried overnight in vacuo to give a white powder, identified by ¹H NMR and elemental analysis as 1,1'-[3,3'-biphenylene-bis-(methylene)]-bis-1,4,8,11-tetraazacyclotetradecane octahydrobromide tetrahydrate (194mg, 50%).
C₃₄H₇₄N₈Br₈O₄ requires; C, 31.46; H, 5.70; N, 8.63; found; C, 31.30; H, 5.68; N, 8.60.

### EXAMPLE 9

### 1,1'-[4,4'-(2,2'-Bipyridine)-bis-(methylene)]-bis-tris-(p-toluenesulphonyl)-1,4,8,11-tetraazacyclotetradecane

A mixture of 4,4'-bis-(bromomethyl)-2,2'-bipyridine [T J Meyer, Inorg. Chem. (1991), 30, 2942-2949], (200mg, 0.57mmol), anhydrous potassium carbonate (314mg, 2.27mmol, 4 eq) and tris-(p-toluenesulfonyl)-1,4,8,11-tetraazacyclotetradecane (774mg, 1.14mmol, 2 eq) in anhydrous acetonitrile (20ml) was stirred at 50°C under argon for 2 hours. The mixture was allowed to cool and dichloromethane (100ml) was added and the resulting solution filtered through celite. The filtrate was evaporated in vacuo to give a yellow glassy solid which was purified by column chromatography on silica gel (3 x 20cm column) using triethylamine/methanol/dichloromethane 1:1:100 v/v as eluent. A glassy white solid was obtained, identified by ¹H NMR as 1,1'-[4,4'-(2,2'-bipyridine)-bis-(methylene)]-bis-tris-(p-toluenesulphonyl)-1,4,8,11-tetraazacyclotetradecane (600mg, 70%).

### Synthesis of Compound K

### 1,1'-[4,4'-(2,2'-Bipyridine)-bis-(methyl)]-bis-1,4,8,11-tetraazacyclotetradecane decahydrobromide pentahydrate

The per-tosylate derivative from above (570mg, 0.38mmol) was dissolved in glacial acetic add (6.5ml). Hydrobromic acid (∼48% w/v, Aldrich, 3.0mmol) was added and the mixture heated to reflux for 24 hours. The resulting dark brown solution was cooled in an ice bath over 2 hours) during which time an off-white precipitate formed. The precipitate was collected by centrifugation and washed with glacial acetic acid (3 x 10ml) followed by diethyl ether (5 x 10ml) and dried overnight in vacuo to give a white powder identified by ¹H NMR and elemental analysis as 1,1'-[4,4'-(2,2'-bipyridine)-bis-(methylene)]-bis-1,4,8,11-tetraazacyclotetradecane decahydrobromide pentahydrate (450mg, 81%).
C₃₂H₇₆N₁₀Br₁₀O₅ requires; C, 25.97; H, 5.17; N, 9.46; found; C, 26.07; H, 4.57; N, 9.47.

### EXAMPLE 10

### 1,1'-[2,9-(1,10-Phenanthroline)-bis-(methylene)]-bis-tris-(p-toluenesulphonyl)-1,4,8,11-tetraazacyclotetradecane

A mixture of 2,9-bis-(bromomethyl)-1,10-phenanthroline [C J Chandler, J. Heterocycl. Chem. (1981), 18, 599-601], (200mg, 0.54mmol), anhydrous potassium carbonate (300mg, 2.17mmol, 4 eq) and tris-(p-toluenesulphonyl)-1,4,8,11-tetraazacyclotetradecane (740mg, 1.09mmol, 2 eq) in anhydrous acetonitrile (20ml) were stirred at 50°C under argon for 3 hours. The mixture was allowed to cool and dichloromethane (100ml) was added and the resulting solution filtered through celite. The filtrate was evaporated in vacuo to give a yellow glassy solid which was purified by column chromatography on silica gel (3 x 20cm column) using triethylamine/methanol/dichloromethane 1:3:100 v/v eluent. A pale yellow solid was obtained, identified by ¹H NMR as 1,1'-[2,9-(1,10-phenanthroline)-bis-(methylene)]-bis-tris-(p-toluenesulphonyl)-1,4,8,11-tetraazacyclotetradecane (575mg, 69%).

### Synthesis of Compound L

### 1,1'-[2,9-(1,10-Phenanthroline)-bis-(methylene)]-bis-1,4,8,11-tetraazacyclotetradecane decahydrobromide trihydrate

The per-tosylated derivative from above (400mg, 0.26mmol) was dissolved in glacial acetic acid (8ml). Hydrobromic acid (∼48% w/v, Aldrich, 3.5ml) was added and the mixture was heated to reflux for 16 hours. The resulting dark brown solution was cooled in an ice bath over 2 hours during which time an off-white precipitate formed. The precipitate was collected by centrifugation then purified by re-precipitation from a mixture of hydrobromic acid (∼48% w/v, 2ml) and water (2ml) with glacial acetic acid (5ml). The white solid was again collected by centrifugation, washed with glacial acetic acid (3 x 10ml) and diethyl ether (4 x 10ml) and finally dried overnight in vacuo to give a white powder, identified by ¹H NMR and elemental analysis as 1,1'-[2,9-(1,10-phenanthroline)-bis-(methylene)]-bis-1,4,8,11-tetraazacyclotetradecane decahydrobromide trihydrate (80mg, 21%).
C₃₄H₇₂N₁₀Br₁₀O₃ requires; C, 27.82; H, 4.94; N, 9.54; found; C, 27.81; H, 4.97; N, 9.17.

### EXAMPLE 11

This compound and corresponding intermediates are described by T A Kaden, Helv. Chim. Acta., (1985), 69, 53-61. An alternative procedure is given below.

### 11,11'-[1,4-Phenylene-bis-(methylene)]-bis-tris-(p-toluenesulphonyl)-1,4,7,11-tetraazacyclotetradecane

A mixture of α,α'-dibromo-p-xylene (249mg, 0.94mmol), anhydrous potassium carbonate (652mg, 4.71mmol, 5 eq) and tris-(p-toluenesulphonyl)-1,4,7,11-tetraazacyclotetradecane [T A Kaden, Helv. Chim. Acta., (1983), 66, 861-870] (1.25g, 1.89mmol, 2 eq) in anhydrous acetonitrile (15ml) was heated at 50°C with stirring under argon for 18 hours. The reaction mixture was allowed to cool and dichloromethane (50ml) was added and the resulting solution filtered through celite. The filtrate was evaporated in vacuo to give a white foam which was purified by column chromatography on silica gel using methanol/dichloromethane (1:40 v/v) as eluent. A white solid was obtained, identified by ¹H NMR as 11,11'-[1,4-phenylene-bis-(methylene)]-bis-tris-(p-toluenesulphonyl)-1,4,7,11-tetraazacyclotetradecane (1.0g, 74%).

### Synthesis of Compound M

### 11,11'-[1,4-Phenylene-bis-(methylene)]-bis-1,4,7,11-tetraazacyclotetradecane octahydrobromide dihydrate

The per-tosylated derivative from above (500mg, 0.35mmol) was dissolved in glacial acetic acid (7ml). Hydrobromic acid (∼48% w/v, 4ml) was added and the resulting mixture heated to reflux for 20 hours. Further glacial acetic acid (10ml) was added and the solution was cooled in an ice bath over 1 hour during which time a white precipitate formed. The solid was collected by centrifugation and washed with glacial acetic acid (2 x 10ml) followed by diethyl ether (4 x 10ml) and dried overnight in vacuo to give a white powder, identified by ¹H NMR and elemental analysis as 11,11'-[1,4-phenylene-bis-(methylene)]-bis-1,4,7,11-tetraazacyclotetradecane octahydrobromide dihydrate (280mg, 67%). C₂₈H₆₆N₈Br₈O₂ requires; C, 28.35; H, 5.61; N, 9.45; found; C, 28.34; H, 5.42; N, 9.02.

### EXAMPLE 12

### 11-[(1,Methylene-4-bromomethylene)-phenylene]-tris-(p-toluenesulphonyl)-1,4,7,11-tetraazacyclotetradecane

A mixture of α,α'-dibromo-p-xylene (3.98g, 15.1mmol, 10 eq), and anhydrous potassium carbonate (417mg, 3.02mmol, 2 eq) in anhydrous acetonitrile (20ml) was heated to 50°C. With rapid stirring a solution of tris-(p-toluenesulphonyl)-1,4,7,11-tetraazacyclotetradecane (1.0g, 1.51mmol) in anhydrous acetonitrile (20ml) was added dropwise over 4 hours. After a further 1 hour the reaction mixture was allowed to cool and the solvent evaporated in vacuo. The residue was purified by column chromatography on silica gel (5 x 20cm), eluting with a gradient of dichloromethane to methanol/dichloromethane 1:20 v/v over 2 litres total elution volume. To the resulting colourless glass was added dry hexane (150ml) and the mixture was heated to reflux then allowed to cool to room temperature. The precipitate which formed was filtered, washed with hexane (3 x 10ml) followed by diethyl ether (20ml) and dried overnight in vacuo to give the title compound as a white powder (710mg, 53%).

### 1,11'-[1,4-Phenylene-bis-(methylene)]-tris-(p-toluenesulphonyl)-1,4,8,11-tetraazacyclotetradecane)-tris-(p-toluenesulphonyl)-1,4,7,11-tetraazacyclotetradecane

A mixture of 11-[(1-methylene-4-bromomethylene)-phenylene]-tris-(p-toluenesulphonyl)-1,4,7,11-tetraazacyclotetradecane (350mg, 0.41mmol), anhydrous potassium carbonate (230mg, 1.66mmol, 4 eq) and tris-(p-toluenesulphonyl)-1,4,8,11-tetraazacyclotetradecane (422mg, 0.62mmol, 1.5 eq) in anhydrous acetonitrile (20ml) were heated with stirring at 50°C under argon for 7 hours. The reaction mixture was allowed to cool and the solvent was evaporated in vacuo. The residue was purified by column chromatography on silica gel (2.5 x 25cm column) using methanol/dichloromethane (1:60 v/v) as eluent, followed by preparative thin layer chromatography on silica gel (eluent methanol/dichloromethane 1:40 v/v, 20 mg/plate) to give a colourless glass, identified by ¹H NMR as the title compound (130mg, 30%).

### Synthesis of Compound N

### 1,11'-[1,4-Phenylene-bis-(methylene)]-1,4,8,11-tetraazacyclotetradecane-1,4,7,11-tetraazacyclotetradecane octahydrobromide hexahydrate

The per-tosylated derivative from above (115mg, 0.08mmol) was dissolved in glacial acetic acid (3ml). Hydrobromic acid (∼48%, Aldrich, 1.3mmol) was added and the mixture was heated to reflux for 48 hour. The resulting dark brown solution was cooled in an ice bath and a white precipitate formed. The solid was collected by centrifugation and washed with glacial acetic acid (3 x 10ml) followed by diethyl ether (5 x 10ml) and dried overnight in vacuo to give a white powder, identified by ¹H NMR and elemental analysis as 1,11'-[1,4-phenylene-bis-(methylene)]-1,4,8,11-tetraazacyclotetradecane-1,4,7,11-tetraazacyclotetradecane octahydrobromide hexahydrate. (71mg, 75%). C₂₉H₇₄N₈Br₈O₆ requires; C, 26.73; H, 5.93; N, 8.91; found; C, 26.50; H, 5.69; N, 9.31.

### EXAMPLE 13

### 1,1'-[2,6-Pyridinebis-(methylene)]-bis-tris-(p-toluenesulphonyl)-1,4,8,11-tetraazacyclotetradecane

A stirred solution of 2,6-bis(bromomethyl)pyridine hydrobromide [M E Haeg, B J Whitlock and H W Whitlock Jr, J. Am. Chem. Soc., (1989), 111, 692], (131mg, 0.378mmol), tris-(p-toluene-sulphonyl)-1,4,8,11-tetraazacyclotetradecane (500mg, 0.75mmol) and potassium carbonate (400mg, 2.88mmol) in anhydrous acetonitrile (15ml) was heated at 80°C for 22 hours under an atmosphere of argon. The reaction mixture was allowed to cool to room temperature and concentrated in vacuo. The residue was purified by column chromatography on silica gel using 3% methanol in dichloromethane as eluent thus affording a pale white solid which was identified by ¹H NMR and FAB-MS as 1,1'-[2,6-pyridinebis-(methylene)]-bis-tris(p-toluene-sulphonyl)-1,4,8,11-tetraacyclotetradecane (500mg, 93%).
Mass spectrum (FAB); m/e (relative intensity); 1428 (M+1, 100), 1272 (35).

### Synthesis of Compound O

### 1,1'-[2,6-Pyridinebis-(methylene)]-bis-1,4,8,11-tetraazacyclotetradecane octahydrobromide tetrahydrate

To a stirred solution of 1,1'-[2,6-pyridinebis-(methylene)]-bis-tris(p-toluenesulphonyl)-1,4,8,11-tetraazacyclotetradecane(500mg,0.35mmol) in acetic acid (16ml) was added 48% hydrobromic acid (12ml) and the solution heated to 110°C for 48 hours during which time a white solid precipitated. The reaction mixture was allowed to cool to room temperature and the solid was filtered off, washed with acetic acid followed by ether and dried in vacuo thus affording a white solid which was identified by ¹H NMR, ¹³C NMR, FAB-MS and elemental analysis as 1,1'-[2,6-pyridinebis-(methylene)]-bis-1,4,8,11-tetraazacyclotetradecane octahydrobromide tetrahydrate (230mg, 65%).
C₂₇H₆₉N₉O₄Br₈ requires; C, 26.50; H, 5.64; N, 10.31; Br, 52.29; found C, 26.91; H, 5.31; N, 10.08; Br, 51.99. Mass spectrum (FAB); m/e (relative intensity); 586 (M+HBr, 48), 584 (M+HBr, 50), 504 (M+1, 100), 201 (60).

### EXAMPLE 14

### 1,1'-[3,5-Pyridine-bis-(methylene)]-bis-tris(p-toluenesulphonyl)-1,4,8,11-tetraazacyclotetradecane

A stirred solution of 3,5-bis(bromomethyl)pyridine hydrobromide (M Momenteau, J Mispelter, B Loock and J M Lhoste, J. Chem. Soc. Perkin Trans. 1, (1985), 61], (131mg, 0.37mmol), tris-(p-toluenesulphonyl)-1,4,8,11-tetraazacyclotetradecane (500mg, 0.755mmol) and potassium carbonate (400mg, 2.88mmol) in anhydrous dimethylformamide (15ml) were heated at 70°C for 21 hours under an atmosphere of argon. The reaction mixture was allowed to cool to room temperature and concentrated in vacuo. The residue was purified by column chromatography on silica gel using 2% methanol in dichloromethane as eluent thus affording a white foamy solid which was identified by ¹H NMR and FAB-MS as 1,1'-[3,5-pyridinebis-(methylene)]-bis-tris(p-toluenesulphonyl)-1,4,8,11-tetraazacyclotetradecane (320mg, 78%).
Mass spectrum (FAB); m/e (relative intensity); 1428 (M+1,100), 1272 (45).

### Synthesis of Compound P

### 1,1'[3,5-Pyridinebis-(methylene)]-bis-1,4,8,11-tetraazacyclotetradecane nonahydrobromide dihydrate

To a stirred solution of 1,1'-[3,5-pyridinebis-(methylene)-bis-tris(p-toluenesulphonyl)-1,4,8,11-tetraazacyclotetradecane (320mg, 0.224mmol) in acetic acid (12ml) was added 48% hydrobromic acid (8ml) and the solution heated to 100°C for 48 hours during which time a white solid precipitated. The reaction mixture was allowed to cool to room temperature and the solid was filtered off, washed with acetic acid followed by ether and dried in vacuo thus affording a white solid which was identified by ¹H NMR, ¹³C NMR, FAB-MS and elemental analysis as 1,1'-[3,5-pyridinebis-(methylene)]-bis-1,4,8,11-tetraazacyclotetradecane nonahydrobromide dihydrate (150mg, 53%).
C₂₇H₆₆N₉O₂Br₉ requires; C, 25.56; H, 5.21; N, 9.94; Br, 56.74; found C, 25.71; H, 5.25; N, 9.76; Br, 56.28. Mass spectrum (FAB); m/e (relative intensity); 586 (M+HBr, 39), 584 (M+HBr, 41), 504 (M+1, 60), 201 (100).

### EXAMPLE 15

### 1,1'-[1,3-Phenylenebis-(methylene)]-bis-tris(p-toluenesulphonyl)-1,4,7,10-tetraazacyclododecane

A stirred solution of α,α'-dibromo-m-xylene (125mg, 0.472mmol), tris-(p-toluenesulphonyl)-1,4,7,10-tetraazacyclododecane [M F Tweedle et al, Inorg. Chem., (1992), 30, 1265], (600mg, 0.945mmol) and potassium carbonate (400mg, 2.88mmol) in anhydrous acetonitrile (15ml) were heated to reflux for 6 hours under an atmosphere of argon. The resulting cloudy white solution was allowed to cool to room temperature and the solids collected by filtration and washed with acetonitrile. The solid residue was dissolved in a mixture of dichloromethane (100ml) and water (15ml). The organic phase was separated and washed with water (15ml), dried (MgSO₄) and concentrated under reduced pressure. The residue was dried in vacuo thus affording a white foamy solid which was identified by ¹H NMR as 1,1'-[1,3-phenylenebis-(methylene)]-bis-tris-(p-toluenesulphonyl)-1,4,7,10-tetraazacyclododecane (330mg, 51%).

### Synthesis of Compound Q

### 1,1'-[1,3-Phenylenebis-(methylene)]-bis-1,4,7,10-tetraazacyclododecane hexahydrobromide

To a stirred solution of 1,1'-[1,3-phenylenebis-(methylene)]-bis-tris-(p-toluenesulfonyl)-1,4,7,10-tetraazacyclododecane (330mg, 0.24mmol) in anhydrous methanel/tetrahydrofuran (1:2, 15ml) was added 3% sodium amalgam (20g) and dibasic sodium phosphate (400mg). The reaction mixture was vigorously stirred under argon at 70°C for 41 hours. The reaction mixture was allowed to cool to room temperature and the supernatent solution was separated from the solids by decantation then concentrated in vacuo. Chloroform (50ml) and water (5ml) were added to the residue and the aqueous phase was extracted with chloroform (3 x 50ml). Concentration of the combined organic fractions afforded quantitatively a viscous oil which was identified by 1H NMR as 1,1'-[1,3-phenylenebis-(methylene)]-bis-1,4,7,10-tetraazacyclododecane.

Into a stirred solution of 1,1'-[1,3-phenylenebis-(methylene)]-bis-1,4,7,10-tetraazacyclododecane in ethanol (20ml, 95%) was bubbled HBr gas for 15 minutes resulting in an immediate white precipitate. The white solid was filtered off, washed with ethanol and ether and immediately dried in vacuo for 48 hours thus affording a white solid which was identified by ¹H NMR, ¹³C NMR, FAB-MS and elemental analysis as 1,1'-[1,3-phenylene-(methylene)]-bis-1,4,7,10-tetraazacyclododecane hexahydrobromide (130mg, 63%).
C₂₇H₅₂N₈Br₆ requires C, 30.92; H, 5.62; N, 12.02; Br, 51.43; found C, 31.09; H, 5.80; N, 11.90; Br, 51.17. Mass spectrum (FAB); m/e (relative intensity); 529 (IM+HBr, 53), 527 (M+HBr, 55), 447 (M+1, 100), 277 (40), 185 (35).

### EXAMPLE 16

### 1,1'-[1,4-Phenylenebis-(methylene)]-bis-tris(p-toluenesulphonyl)-1,4,7,10-tetraazacyclododecane

A stirred solution of α,α'-dibromo-p-xylene (99mg, 0.374mmol), tris-(p-toluenesulphonyl)-1,4,7,10-tetraazacyclododecane (475mg, 0.748mmol) and potassium carbonate (320mg, 2.24mmol) in anhydrous acetonitrile (15ml) was heated at reflux for 14 hours under an atmosphere of argon. The resulting cloudy white solution was allowed to cool to room temperature and the solids collected by filtration and washed with acetonitrile. The solid residue was dissolved in a mixture of dichloromethane (120ml) and water (15ml). The organic phase was separated and washed with water (15ml), dried (MgSO₄) and concentrated under reduced pressure. The residue was dried in vacuo thus affording a white solid which was identified by ¹H NMR as 1,1'-[1,4-phenylenebis-(methylene)]-bis-tris-(p-toluenesulphonyl)-1,4,7,10-tetraazacyclododecane (360mg, 70%).
Mass spectrum (FAB); m/e (relative intensity); 1371 (M+1, 12), 1217 (8).

### Synthesis of Compound R

### 1,1'-[1,4-Phenylenebis-(methylene)]-bis-1,4,7,10-tetraazacyclododecane hexahydrobromide

To a stirred solution of 1,1'-[1,4-phenylenebis-(methylene)-bis-tris(p-toluenesulphonyl)-1,4,7,10-tetraazacyclododecane (360mg, 0.262mmol) in anhydrous methanol/dimethylsulphoxide (1:5, 18ml) was added 3% sodium amalgam (23g) and dibasic sodium phosphate (400mg). The reaction mixture was vigorously stirred under argon at 100°C for 4 hours then allowed to cool to room temperature and the supernatent solution was separated from the solids by decantation and concentrated in vacuo. Chloroform (50ml) and water (5ml) were added to the residue and the aqueous phase was extracted with chloroform (3x 50ml). Concentration of the combined organic fractions afforded quantitatively a foamy white solid which was identified by ¹H NMR as 1,1'-[1,4-phenylenebis-(methylene)]-bis-1,4,7,10-tetraazacyclododecane.

Into a stirred solution of 1,1'-[1,4-phenylenebis-(methylene)]-bis-1,4,7,10-tetraazacyclododecane in ethanol (15ml, 95%) was bubbled HBr gas for 15 minutes resulting in an immediate white precipitate. The white solid was filtered off, washed with ethanol and ether and immediately dried in vacuo for 48 hours thus affording a white solid which was identified by ¹H NMR, ¹³C NMR, FAB-MS and elemental analysis as 1,1'-[1,4-phenylenebis-(methylene)]-bis-1,4,7,10-tetraazacyclododecane hexahydrobromide (115mg, 47%).
C₂₇ H₅₂N₈Br₆ requires C, 30.92; H, 5.62; N, 12.02; Br 51.43, found C, 30.90; H, 5.83; N, 11.83; Br, 51.19. Mass spectrum (FAB); m/e (relative intensity); 529 (M+HBr, 40), 527 (M+HBr, 40), 447 (M+1, 58), 185 (100).

### EXAMPLE 17

### Tetraethyl-[1,4-phenylene-bis-(methylene)]-bis-malonate

A solution of diethyl malonate (5.0g, 0.03mole) in dry DMF (10ml) was added dropwise with stirring, under Argon to NaH (95%, 0.95g, 1.2 eq) in dry DMF (10ml) at 0.5°C. When the addition was complete the solution was stirred at room temperature for 1 hour. To this solution was added dropwise α,α'-dibromo-p-xylene (4.12g, 0.016mol) in dry DMF (30ml) and the mixture was heated at 55°C for a further 2 hours. The solvent was evaporated in vacuo and the residue was taken up in ethyl acetate (100ml) and washed with 0.1N HCl (50ml); saturated aqueous sodium bicarbonate solution (50ml) and brine (50ml), then dried (Na₂SO₄) and evaporated under reduced pressure to give the crude product as a pale yellow oil. Column chromatography [silica gel, ether/hexane (1/3)] was used to isolate a white solid identified by ¹H NMR as tetraethyl-[1,4-phenylene-bis-(methylene)]-bis-malonate (2.6g, 40%).

### 6,6'-[1,4-Phenylene-bis-(methylene)]-bis-1,4,8,11-tetraazacyclotetradecane-5,7-dione

To a stirred solution of tetraethyl-[1,4-phenylene-bis-(methylene)]-bis-malonate (2.57g, 6.1mmol) in absolute ethanol (100ml), under argon, was added dropwise a solution of 1,4,8,11-tetraazaundecane (1.95g, 12.2mmol) in absolute ethanol (50ml). When the addition was complete the solution was heated to reflux for 20 days during which time a white solid precipitated. The mixture was allowed to cool and the solid was filtered off, washed with ethanol (10ml) and dried in vacuo giving a white solid identified by ¹H NMR as 6,6'-[1,4-phenylene-bis-(methylene)]-bis-1,4,8,11-tetraazacyclotetradecane-5,7-dione (138mg, 4%).

### Synthesis of Compound S

### 6,6'-[1,4-Phenylene-bis-(methylene)]-bis-1,4,8,11-tetraazacyclotetradecane octahydrobromide dihydrate

To a stirred solution of 6,6'-[1,4-phenylene-bis-(methylene)]-bis-1,4,8,11-tetraazacyclotetradecane-5,7-dione (64mg, 0.12mmol) in anhydrous THF (10ml) under an atmosphere of dry argon was added Borane. THF complex (1.0M solution in THF, 5ml) dropwise, and the mixture was heated to reflux overnight. The mixture was allowed to cool and the excess Borane destroyed by addition of anhydrous methanol (50ml) and evaporated (repeated three times). The white residue was dissolved in glacial acetic acid (2.0ml) and hydrobromic acid (Aldrich 48% aqueous, 1.5ml) was added. The mixture was heated at 110°C with stirring for 1 hour during which time a white amorphous solid precipitated. On cooling, a further portion of acetic acid (2ml) was added and the solids were filtered off, washed with acetic acid (2ml) and then ether (10ml) giving 6,6'-[1,4-phenylene-bis-(methylene)]-bis-1,4,8,11-tetraazacyclotetradecane octahydrobromide dihydrate (45mg, 35%).
C₂₈H₆₆N₈Br₈O₂ requires; C, 28.33; H, 5.56; N, 9.44; found C, 27.98; H, 5.57; N, 8.82. Mass spectrum (FAB); m/e (relative intensity); 585 (M+HBr, 53), 583 (M+HBr, 58), 504 (M+1, 100), 331 (20), 305 (40).

### EXAMPLE 18

### 1,1'-[2,5-Thiophene-bis-(methylene)]-bis-tris-(p-toluenesulphonyl)-1,4,8,11-tetraazacyclotetradecane

To a solution of tris-p-toluenesulphonyl-1,4,8,11-tetraazacyclotetradecane monohydrate (1.0g, 1.5mmol) and potassium carbonate (300mg, 2.2mmol) in acetonitrile (20ml) was added 2,5-dichloromethyl thiophene [J M Griffing, L F Salisbury, J. Am. Chem. Soc., (1948), 70, 3416-3419], (137mg, 0.76mmol) and the mixture was heated to reflux overnight with rapid stirring. The mixture was allowed to cool and the solid was filtered off. The filtrate was evaporated in vacuo and the residue partitioned between methylene chloride (50ml) and water (25ml). The organic layer was separated, dried (Na₂SO₄) and evaporated in vacuo to give the crude product as a light brown solid. Column chromatography [silica gel; methylene chloride/methanol(40/1)] was used to isolate a white solid identified by ¹H NMR and FAB-MS as 1,1'-[2,5-thiophene-bis-(methylene)]-bis-tris-(p-toluenesulphonyl)-1,4,8,11-tetraazacyclotetradecane (315mg, 29%).
Mass spectrum (FAB); M/e (relative intensity); 1434 (M+1, 49), 1277 (31), 772 (100), 616 (30), 508 (24).

### Synthesis of Compound T

### 1,1'-[2,5-Thiophenebis-(methylene)]-bis-1,4,8,11-tetraazacyclotetradecane octahydrobromide

To a solution of 1,1'-[2,5-thiophene-bis-(methylene)]-bis-tris-(p-toluenesulphonyl)-1,4,8,11-tetraazacyclotetradecane (177mg, 0.12mmol) in acetic acid (6ml) was added hydrobromic acid (Aldrich 48% aqueous, 4ml) and the mixture was heated to a reflux with stirring for 16 hours during which time a light brown solid precipitated from a dark brown solution. On cooling, a further portion of acetic acid was added (10ml) and the solids were filtered off, washed with acetic acid (10ml) and ether (20ml) and dried in vacuo giving a white solid identified by ¹H NMR and FAB-MS as 1,1'-[2,5-thiophene-bis-(methylene)-bis-1,4,8,11-tetraazacyclotetradecane octahydrobromide (82mg, 97%).
Mass spectrum (FAB); m/e (relative intensity); 591 (M+HBr, 26), 589 (M+Br, 26), 509 (M+1, 22) 311 (23), 201 (71), 185 (100).

### EXAMPLE 19

### 1,1'-[1,4-Phenylene-bis-(methylene)]-bis-tris-(N-acetyl)-1,4,8,11-tetraazacyclotetradecane

1,1'-[1,4-Phenylenebis-(methylene)-bis-1,4,8,11-tetraazacyclotetradecane (200mg, 0.39mmol) was stirred in acetic anhydride (5.0ml) at 55°C for 18 hours. The solution was cooled and ether (100ml) was added precipitating a pale yellow solid. The solid was filtered off then dissolved in methylene chloride (100ml) and washed with water (50ml). The organic layer was dried (Na₂SO₄) and evaporated in vacuo giving 1,1'-[phenylenebis-(methylene)-bis-(tris-N-acetyl)-1,4,8,11-tetraazacyclotetradecane (118mg, 40%) as a white amorphous solid. This was used without further purification.

### Synthesis of Compound U

### 1,1'-[1,4-Phenylene-bis-(methylene)-bis-(tris-N-ethyl)-1,4,8,11-tetraazacyclotetradecane octahydrobromide octahydrate

To a solution of 1,1'-[1,4-phenylenebis-(methylene)-bis-(tris-N-acetyl)-1,4,8,11-tetraazacyclotetradecane (115mg, 0.15mmol) in dry tetrahydrofuran (25ml) was added borane-tetrahydrofuran complex (1.0M solution in THF, Aldrich, 5ml) and the mixture was heated to reflux overnight with rapid stirring. The mixture was allowed to cool and the excess borane destroyed by addition of anhydrous methanol (50ml) and evaporation (repeated three times). To the white residue dissolved in glacial acetic acid (2.5ml) was added hydrobromic acid (Aldrich 48% aqueous, 1.5ml). The mixture was heated to reflux with stirring for 18 hours during which time a white amorphous solid precipitated from a dark brown solution. On cooling, a further portion of acetic acid was added (5ml) and the solids were filtered off, washed with acetic acid (5ml) and then ether (20ml) giving a white powder identified by ¹H NMR and elemental analaysis as 1,1'-[1,4-phenylenebis-(methylene)]-bis-tris-(N-ethyl)-1,4,8,11-tetraazacyclotetradecane octahydrobromide octahydrate (145mg, 73%).
C₄₀H₁₀₂N₈Br₈O₈ requires; C, 32,83; H, 7.03; N, 7.66; Br, 43.71; found; C, 32.74; H, 6.71; N, 7.60; Br, 43.93.

### EXAMPLE 20

### α,α'-Dibromo-2,4-dimethylquinoline

To a solution of 2,4-dimethylquinoline (0.3g, 1.9mmol) and benzoylperoxide (47mg, 0.10 eq) in carbon tetrachloride (20ml) was added N-bromosuccinimide (0.68g, 2.0 eq) and the mixture was heated to reflux for 2 hours during which time a white solid precipitated. The mixture was filtered hot (to remove the succinimide by-product) and then evaporated in vacuo to give the crude product as a brown solid. Column chromatography [silica gel, methylene chloride] was used to isolate the pure product as a white solid (95mg, 16%).

### 1,1'-[2,4-Quinoline-bis-(methylene)]-bis-tris-(diethylphosphoryl)-1,4,8,11-tetraazacyclotetradecane

To a solution of tris-(diethylphosphoryl)-1,4,8,11-tetraazacyclotetradecane (0.51g, 0.83mmol) and potassium carbonate (0.29g, 1.7mmol) in acetonitrile (20ml) was added α,α'-dibromo-2,4-dimethylquinoline (0.13g, 0.41mmol) and the mixture was heated to reflux overnight with rapid stirring. The mixture was allowed to cool and the solid was filtered off. The filtrate was evaporated and the residue partitioned between methylene chloride (100ml) and water (50ml). The organic layer was separated, dried (Na₂SO₄) and evaporated in vacuo to give the crude product as a brown gummy solid. Column chromatography [silica gel, methylene chloride/methanol (97/3)] was used to isolate a white solid identified by ¹H NMR as 1,1'-[2,4-quinoline-bis-(methylene)]-bis-tris(diethylphosphoryl)-1,4,8,11-tetraazacyclotetradecane (210mg, 72%).

### Synthesis of Compound V

### 1,1'-[2,4-Quinoline-bis-(methylene)]-bis-1,4,8,11-tetraazacyclotetradecane nonahydrobromide

To a solution of 1,1'-[2,4-quinoline-bis-(methylene)]-bis-tris-(diethylphosphoryl)-1,4,8,11-tetraazacyclotetradecane (170mg, 0.13mmol) in acetic acid (2.5ml) was added hydrobromic acid (Aldrich 48% aqueous, 1.5ml) and the mixture was stirred at room temperature for 5 hours during which time a white solid precipitated from a brown solution. The solid were filtered off, washed with acetic acid (10ml) and ether (20ml) giving a white solid identified by ¹H NMR and FAB-MS as 1,1'-[2,4-quinoline-bis-(methylene)] -bis-1,4,8,11-tetraazacyclotetradecane nonahydrobromide (0.15g, 94%).
Mass spectrum (FAB; m/e (relative intensity); 636 (M+Br, 11), 634 (M+Br, 11), 554 (M+1, 13), 356 (14), 229 (37), 201 (100).

The compounds of the invention were tested in a screen by the MTT method (J Virol Methods 120: 309-321 [1988]). MT-4 cells (2.5 x 10⁴/well) were challenged with HIV-1 (HTLV-IIIB) or HIV-2 (LAV-2 ROD) at a concentration of 100 CCID₅₀ and incubated in the presence of various concentrations of the test compounds, which were added immediately after challenge with the virus. After 5 days culture at 37°C in a CO₂ incubator, the number of viable cells was assessed by the MTT (tetrazolium) method. Antiviral activity and cytotoxicity of the compounds are expressed in the table below as IC₅₀ (µg/ml) and CC₅₀ (µg/ml), respectively. The potential therapeutic usefulness was assessed by calculating a Selectivity Index (SI) corresponding to the ratio of CC₅₀ to IC₅₀. A control test was performed using the known anti-HIV treatment AZT.

In Table 1 below, the compounds screened were:
- AZT :: known anti-HIV compound
- A :: 1,1'-[1,3-phenylenebis(methylene)]bis-1,4,8,11-tetraazacyclotetradecane
- B :: 1,1'-[1,4-phenylenebis(methylene)]bis-1,4,8,11-tetraazacyclotetradecane
- C :: 1,1'-[5-nitro-1,3-phenylenebis-(methylene)]-bis-1,4,8,11tetraazacyclotetradecane
- D :: 1,1'-[2,3,5,6-tetrafluoro-1,3-phenylene-bis-(methylene)]-bis-1,4,8,11-tetraazacyclotetradecane
- E :: 1,1'-[1,4-naphthylenebis(methylene)]bis-1,4,8,11-tetraaza-cyclotetradecane
- F-V :: See preceding preparative Examples.
- W :: 1,1'-[2,5-dimethyl-1,4-phenylenebis-(methylene)]-bis-1,4,8,11-tetraazacyclotetradecane
- X :: 1,1'-[2,5-dichloro-1,4-phenylenebis-(methylene)]-bis-1,4,8,11-tetraazacyclotetradecane
- Y :: 1,1'-[2-bromo-1,4-phenylenebis-(methylene)]-bis-1,4,8,11-tetraazacyclotetradecane
- Z :: 1,1'-[6-phenyl-2,4-pyridinebis-(methylene)]-bis-1,4,8,11-tetraazacyclotetradecane

**TABLE 1**

| | HIV-1 (III₈) | | | HIV-2 (ROD) | | |
|---|---|---|---|---|---|---|
| CMPD | IC₅₀ µg/ml | CC₅₀ µg/ml | SI | IC₅₀ µg/ml | CC₅₀ µg/ml | SI |
| AZT Comparison | <0.008 | >1 | >125 | - | - | - |
| A | 0.03 | >500 | >1.6x10⁴ | <0.01 | >500 | >5x10⁴ |
| B | 0.006 | >500 | >8.3x10⁴ | <0.01 | >500 | >5x10⁴ |
| C | 0.05 | 55 | 1100 | 0.07 | 55 | 756 |
| D | 0.01 | 60 | 6000 | 0.01 | 60 | 6000 |
| E | 0.07 | 71 | 1014 | 0.05 | 71 | 1420 |
| F | 0.0026 | >200 | >7.6x10⁴ | 0.0019 | >200 | >1x10⁵ |
| G | 0.018 | >200 | >1.1x10⁴ | 0.027 | >200 | >7.4x10³ |
| H | 0.5 | 352 | 704 | 18 | 352 | 20 |
| I | 2.4 | 305 | 127 | 24 | 305 | 12.7 |
| J | 0.16 | >200 | >1250 | 0.22 | >200 | >900 |
| K | 0.38 | 117 | 300 | 0.35 | 117 | 334 |
| L | 0.29 | >200 | >690 | 0.32 | >200 | >625 |
| M | 0.03 | >500 | >1.6x10⁴ | 0.07 | >500 | >7.1x10³ |
| N | 0.01 | >500 | >5x10⁴ | 0.07 | >500 | >7.1x10³ |
| O | 0.03 | >500 | >1.6x10⁴ | 0.08 | >500 | >6.2x10³ |
| P | 0.04 | >500 | >1.2x10⁴ | 0.09 | >500 | >5.5x10³ |
| Q | 0.07 | 19 | 271 | 0.5 | 19 | 38 |
| R | 0.3 | 51 | 170 | 2.2 | 51 | 23 |
| S | 0.6 | 482 | 803 | 0.8 | 482 | 603 |
| T | 0.01 | >300 | >5.0x10⁴ | 0.02 | >500 | >2.5x10⁴ |
| U | 11.4 | >500 | >44 | 21.3 | >500 | >24 |
| V | 4.5 | >500 | >110 | 5.1 | >500 | >98 |
| W | 0.0076 | >250 | >3.2x10⁴ | 0.0013 | >250 | >1.9x10⁵ |
| X | 0.0131 | 71.87 | 5461 | 0.0030 | 72.66 | 2.4x10⁴ |
| Y | 0.0075 | >250 | >3.2x10⁴ | 0.0043 | >250 | >5.7x10⁴ |
| Z | 0.0489 | >250 | 5112 | 0.0246 | >250 | 1.0x10⁴ |

It can readily be seen that the compounds according to the invention are highly active against HIV-1 and -2, with low toxicity, in the in vitro tests used.

The compound B, which is the most preferred compound of the invention, was further tested for antiviral effects on different laboratory strains of HIV-1 in MT-4 cells, using the MIT assay. Compound B was found to have an IC₅₀ in the range of 2-3ng/ml against IIIb, RF, HE and NDK strains, showing that its high activity is remarkably strain-independent.

T4-lymphocytes and monocytes are targets for HIV-1 infection in vivo. The following test method showed that compound B inhibits virus replication also in primary T4 cells and primary monocytes in culture.

Primary T4 lymphocytes were purified from human spleens obtained from healthy donors by using a commercial kit ("Lympho-Kwik") which combines reaction of cells with specific monoclonal antibodies and density gradient centrifugation to separate the cells. Preparations obtained by this procedure contained 60-80% CD4 positive cells as analysed by FACS. Cells were stimulated with 2µg/ml PHA for 24 hours. Then they were spun down and infected with HIV-1, strain IIIb, by suspending the cells 10-fold concentrated in virus solution. Adsorption was allowed for 2 hours at 37°C. The inoculum was removed by centrifugation and the cells were re-suspended at their original concentration in fresh culture medium containing IL-2 (40 IE/ml). Test compound was added after stimulation and virus adsorption. Every 3 to 4 days post infection half of the supernatant of the infected cultures was removed and replaced by fresh medium containing the test compound at the particular concentration, The concentration of viral p24 antigen was determined in the supernatants by means of a commercial ELISA kit (Coulter) and served as a parameter for virus production. Compound B does not interfere with the p24 Elisa test (highest concentration tested: 100µg/ml).

Mononuclear cells were isolated from healthy, HIV-negative donors using Ficoll density separation. Cells (4x10⁶/ml) were incubated for 5 days in 48 well plates (Costar) in monocyte medium, consisting of RPMI1640, supplemented with 20% ECS and 10% human serum. On day 5 non-adherent cells were washed out four times with warm PBS containing 2% human serum. Preparations obtained by this procedure were >95% positive for non-specific esterase (Sigma) and cell viability (as determined by trypan blue exclusion) was always >95%.

The monocytotropic strain of HIV-1, BaL, was used for the infection of these monocyte preparations (Pemo et al, J Exp Med, 169, 933, 1989).

Adherent monocytes were exposed to 50µg/well of a 1:30 dilution of HIV-1, BaL for 30 minutes subsequently, monocyte medium was added to 1ml/well. Adsorption was allowed for 24 hours at 37°C. Then, the wells were washed twice in order to remove excess virus and were cultivated in the presence of different drug concentrations. Thus, test compounds were added after adsorption. Every 3 to 4 days post infection the supernatant of the infected cultures was removed and replaced by fresh medium containing the test compound at the particular concentration. The concentration of viral p24 antigen was determined as described above.

IC₅₀ and IC₉₀ values were calculated by comparing the p24 antigen concentrations in supernatants of treated, infected cells and untreated, infected cells at days 11 and 14 post infection.

Table 2 shows that Compound B is a potent inhibitor of HIV-I replication in both primary cell types, with IC₉₀ values of 1-2ng/ml. At the highest concentration tested, 100ng/ml, no cytotoxicity was observed.

**TABLE 2**

| | | | | | |
|---|---|---|---|---|---|
| Activity of Compound B and AZT against HIV-1, IIIb, replication in primary T4 lymphocytes and against HIV-1, BaL, replication in primary monocytes. | | | | | |

| Compound | Cell Type | IC₅₀ (µg/ml) | | IC₉₀ (µg/ml) | |
|---|---|---|---|---|---|
| | | day 11 | day 14 | day 11 | day 14 |
| B | Lymphocytes | <0.001 | <0.001 | <0.001 | 0.0010 |
| AZT | Lymphocytes | 0.00045 | 0.00043 | 0.0022 | 0.0011 |
| B | Monocytes | <0.001 | 0.0011 | 0.0019 | 0.0021 |
| AZT | Monocytes | 0.0010 | 0.0010 | 0.0015 | 0.0017 |

Using the same methods, it was also shown that Compound B was a strong inhibitor of viral replication in primary T4 cells infected with low-passage primary clinical isolates of HIV-1 from three different geographical locations (K31, Zaire, D370, California, and K6/2, Germany).

The low cytotoxicity of Compound B was also shown by incubation of exponentially growing cells with Compound B or with AZT and determining cell numbers 2, 3 and 4 days after seeding. Compound B did not inhibit growth of MT4, MOLT4, HUT78, Jurkat cells (all T cell lines) nor the growth of the monocylic U937 cell line at concentrations below 300µg/ml. With the exception of the HUT78 cells, AZT was in all cases more cytotoxic than Compound B with TC₅₀ values (µg/ml) of 23, 37, 184 and 5 for MT4, MOLT4, Jurkat and U937 respectively.

In contrast to HIV-protease inhibitors, the compounds of the invention do not block virus production from chronically infected cells. indicating that the antiviral target is in the early part of the infection process, before, or at, integration of the provirus. To pinpoint the stage at which the compounds interact with the HIV replicative cycle, a time-of-addition experiment was carried out on MT4 cells infected with HIV-1 strain IIIb at high virus multiplicity to ensure that the virus replicative steps would be synchronised in the whole cell populations. Test compounds were added 1, 2, 3, ..... 22, 23, 24 hours after infection, and viral p24 antigen production determined 29 hours after infection.

Depending on the stage at which compounds interact and the need for intracellular metabolism, addition of the compounds could be delayed for n hours without loss of activity. Dextran sulphate, which acts at the virus adsorption step, must be added together with the virus (n=0) to be active. For AZT, which, following its intercellular phosphorylation, acts at the reverse transcriptase step, addition to the cells could be delayed until ca 4 hours (n=4) after infection. For the TIBO derivative (R82913), which does not need intracellular transformation before it can interact with reverse transcriptase the addition could be delayed by another 2 hours (n=6). The protease inhibitor Ro31-8959 which interacts with a late event in the virus cycle (assembly of mature virus) was still effective if added as late as 12 hours after infection (n=12). From the time-of-addition experiment appeared that for Compound B, n = 1 or 2, so that the compound must interact with a process following virus absorption but preceding reverse transcription, for example, virus-cell fusion and/or uncoating.

To obtain further evidence for the inhibitory effect of Compound B on HIV uncoating (or fusion), experiments were designed whereby the viral RNA harvested from cells that had just been infected was monitored for its sensitivity to degradation by RNase. It was reasoned that if uncoating (fusion) was hampered, the viral capsid (or envelope) proteins would remain associated with the viral RNA genome and thus the RNA should be protected against RNase attack. When MT4 cells were exposed to radiolabelled viral particles at a very high multiple of infection and then treated with different concentrations of Compound B, viral RNA harvested from the cells 4 hours after infection showed resistance to degradation by RNaseA. Viral RNA harvested from HIV-infected cells treated with other anti-HIV agents (ie AZT, DDI, R82913, or Ro31-8959) did not show this increased resistance to degradation by RNase.

In addition, Compound B was also found to inhibit fusion, which is the mechanism by which viruses enter cells and by which virus or infectious material is transmitted from cell to cell. Syncytium formation between chronically infected cells and uninfected cells reflects the gp120/41 mediated fusion process of viral entry. The syncytium inhibition assay (Baba et al, J AIDS 3 493, 1990)) using HIV-1 IIIb infected HUT78 cells with MOLT4 cells indicates that Compound B is at least as potent as dextran sulphate in inhibition of fusion. The concentrations required (approximately 1µg/ml) are considerably higher than the antiviral IC₅₀ values, but are well below cytotoxicity levels.

These results strongly indicate that the compounds of the invention inhibit primarily the uncoating step and also to some extent the fusion step of the viral replicative cycle. This is a unique mode of action for anti-HIV agents, and the involvement of two distinct target steps makes it less likely that resistance to the drug will develop rapidly in treated patients.

Although no suitable animal models exist for the testing of in vivo efficacy of anti-HIV agents, testing of drug serum levels in the rabbit was carried out, and after sc administration of 10mg/kg of Compound B, samples of rabbit serum were taken. Measurement of anti-HIV activity in the serum showed levels of the drug exceeding the in vitro IC₅₀ level by a factor of a hundred for at least 6 hours after administration. This indicates that the compound would have anti-HIV activity in humans or an animal susceptible to infection by HIV.

The compounds of Formula I are therefore useful for the treatment and/or prophylaxis of HIV infection, alone or in combination with other active agents. The appropriate dosage will, of course, vary depending upon, for example, the compound of Formula I employed, the host, the mode of administration and the nature and severity of the conditions being treated. However, in general, satisfactory results in humans are indicated to be obtained at daily dosages from about 0.01-20mg/kg of body weight. An indicated daily dosage in humans is in the range from about 0.7mg to about 1400mg of a compound of Formula I conveniently administered for example in divided doses up to four times a day.

The compounds of Formula I may be administered by any conventional route, particularly enterally, preferably orally, eg in the form of tablets or capsules or in liquid form, eg as a syrup; or parenterally, eg in the form of solutions or suspensions for iv or sc administration.

Compound B is the preferred compound of Formula I. In view of its activity in the test methods as described above, it is indicated that Compound B may be administered to humans at daily dosages of from 2 to 200mg, preferably 10 to 70mg, by parenteral administration, eg by subcutaneous injection.

The compounds of Formula I may be administered in free base form or in pharmaceutically acceptable acid addition salt or metal complex form. Such salts and complexes may be prepared in conventional manner as described in the Examples, and exhibit the same order of activity as the free bases. Pharmaceutical compositions containing compounds of Formula I may be manufactured in conventional manner. Unit dosage forms contain for example from about 0.5mg to about 100mg of a compound of Formula I in free base or pharmaceutically acceptable acid addition salt form.

## Claims

1. A pharmaceutical composition comprising as an active ingredient a compound of formula I,
Z-R-A-R'-Y (I)
in which Z and Y are cyclic polyamine moieties having from 9 to 32 ring members and from 3 to 8 amine nitrogens in the ring spaced by 2 or more carbon atoms from each other,
A is an aromatic or heteroaromatic moiety, unsubstituted or substituted with alkyl, sulfhydryl, alkylthio, hydroxyl, alkoxyl, amino, nitro, halogen, carboxy, carboxamidyl, or sulfonic acid;
each of R and R' is independently alkylene of 1-12 atoms optionally containing one or more heteroatoms selected from N, S and O and which is optionally unsaturated or polyunsaturated,
the groups being linked to nitrogen atoms in Z and Y,
or a non-toxic acid addition salt or metal complex thereof, in admixture or association with a pharmaceutically acceptable diluent or carrier.

2. A composition according to claim 1, wherein Z and Y are not identical.

3. A composition according to claim 1, wherein R and R' are saturated alkylene.

4. A composition according to claim 3, wherein at least one of R and R' is other than methylene.

5. A composition according to claim 1, wherein R and R' are identical.

6. A composition according to claim 1 or 2, wherein in the compound of formula I, each moiety Z and Y has 10-15 ring members and 3-6 amine nitrogens in the ring.

7. A composition according to claim 1, wherein A is a phenylene, biphenylene, pyridylene, bipyridylene, thiophenylene or napthylene which is substituted or unsubstituted.

8. A composition according to claim 6, wherein, in the compound of formula I, each of Z and Y has 14 ring members and 4 amine nitrogens in the ring.

9. A composition according to claim 1, wherein one active ingredient is
1,1'-[1,4-phenylene-bis-(methylene)]-1,4,8,11-tetraazacyclotetradecane-1,5,9,13-tetraazacyclohexadecane octahydrobromide;
1,11'-[1,4-phenylene-bix-(methylene)]-1,4,8,11-tetraazacyclotetradecane-1,4,7,11-tetraazacyclotetradecane octahydrobromide hexahydrate;
1,1'-[3,3'-biphenylenebis(methylene)]bis-1,4,8,11-tetraazacyclotetradecane;
11,11'-[1,4-phenylenebis(methylene)]bis-1,4,7,11-tetraazacyclotetradecane;
1,11'-[1,4-phenylenebis(methylene)]-1,4,8,11-tetrazacyclotetradecane-1,4,7,11-tetraazacyclotetradecane;
1,1'-[2,6-pyridinebis(methylene)]bis-1,4,8,11-tetraazacyclotetradecane;
1,1-[3,5-pyridinebis(methylene)]bis-1,4,8,11-tetraazacyclotetradecane;
1,1'-[2,5-thiophenebis(methylene)]bis-1,4,8,11-tetraazacyclotetradecane;
1,1'-[4,4'-(2,2'-bipyridine)bis(methylene)]bis-1,4,8,11-tetraazacyclotetradecane;
1,1'-[1,3-phenylenebis(methylene)]bis-1,4,7,10-tetraazacyclotetradecane;
1,1'-[1,4-phenylenebis(methylene)]bis-1,4,7,10-tetraazacyclotetradecane;
1,1'-[2,3,5,6-tetrafluoro-1,4-phenylenebis(methylene)]bis-1,4,8,11-tetraazacyclotetradecane;
1,1'-[1,4-naphthylenebis(methylene)]bis-1,4,8,11-tetraazacyclotetradecane;
1,1'-[1,3-phenylenebis(methylene)]bis-1,5,9-triaazacyclododecane;
1,1'-[1,4-phenylenebis(methylene)]bis-1,5,9-triaazacyclododecane;
1,1'-[2,5-dimethyl-1,4-phenylenebis(methylene)]bis-1,4,8,11-tetraazacyclotetradecane;
1,1'-[2,5-dichloro-1,4-phenylenebis(methylene)]bis-1,4,8,11-tetraazacyclotetradecane;
1,1 '-[2-bromo-1,4-phenylenebis(methylene)]bis-1,4,8,11-tetraazacyclotetradecane; or
1,1 '-[6-phenyl-2,4-pyridinebis(methylene)]bis-1,4,8,11-tetraazacyclotetradecane;
or an acid addition salt form, or metal complex thereof.

10. A composition according to any one of the preceding claims, in unit dosage form.

11. A compound of formula Ia,
Z―R―A'―R'―Y (Ia)
and its addition salts wherein Z and Y, and R and R' are as defined in claim 1, and A' is as A is defined in claim 1, with the proviso that when Z and Y are 14-membered tetraaza rings, and R and R' are methylene, A' is not unsubstituted phenylene.

12. A compound according to claim 11, wherein Y and Z are not identical.

13. The compound of claim 11, which is
1,1'-[1,4-phenylene-bis-(methylene)]-1,4,8,11-tetraazacyclotetradecane-1,5,9,13-tetraazacyclohexadecane octahydrobromide;
1,11'-[1,4-phenylene-bix-(methylene)]-1,4,8,11-tetraazacyclotetradecane-1,4,7,11-tetraazacyclotetradecane octahydrobromide hexahydrate;
1,1'-[2,3,5,6-tetrafluoro-1,4-phenylenebis(methylene)]bis-1,4,8,11-tetraazacyclotetradecane;
1,1'-[1,4-naphthylenebis(methylene)]bis-1,4,8,11-tetraazacyclotetradecane;
1,1'-[1,3-phenylenebis(methylene)]bis-1,5,9-tetraazacyclodecane;
1,1'-[1,4-phenylenebis(methylene)]bis-1,5,9-tetraazacyclodecane;
1,1'-[3,3'-biphenylenebis(methylene)]bis-1,4,8,11-tetraazacyclotetradecane;
11,11'-[1,4-phenylenebis(methylene)]bis-1,4,7,11-tetrazacyclotetradecane;
1,1'-[2,6-pyridinebis(methylene)]bis-1,4,8,11-tetraazacyclotetradecane;
1,1'-[3,5-pyridinebis(methylene)]bis-1,4,8,11-tetraazacyclotetradecane;
1,1'-[2,5-thiophenebis(methylene)]bis-1,4,8,11-tetraazacyclotetradecane;
1,1'-[4,4'-(2,2'-bipyridine)-bis-(methylene)]bis-1,4,8,11-tetaazacyclotetradecane;
1,1'-[1,3-phenylenebis(methylene)]bis-1,4,7,10-tetraazacyclotetradecane;
1,1'-[1,4-phenylenebis(methylene)]bis-1,4,7,10-tetraazacyclotetradecane;
1,1'-[2,5-dimethyl-1,4-phenylenebis(methylene)]bis-1,4,7,11-tetraazacyclotetradecane;
1,1'-[2,5-dichloro-1,4-phenylenebis(methylene)]bis-1,4,7,11-tetraazacyclotetradecane;
1,1'-[2-bromo-1,4-phenylenebis(methylene)]bis-1,4,8,11-tetraazacyclotetradecane; or
1,1'-[6-phenyl-2,4-pyridinebis(methylene)]bis-1,4,8,11-tetraazacyclotetradecane
including the acid addition salt forms.

14. A method for the production of a compound according to claim 11, comprising nucleophilic attack by cyclic polyamines Z' and Y' each being the amine corresponding to the substituent Z or Y as defined in claim 1 and having a single unprotected ring amine nitrogen, all other ring amine nitrogens being protected, on a compound of formula II,
X-CH₂―A'―CH₂―X (II)
wherein X is an active substituent which can be displaced by the unprotected amine nitrogens of polyamines Z' and Y',
and subsequently deprotecting the ring amine nitrogens.

15. A method according to claim 14, wherein the nucleophilic attack takes place in the presence of a solvent and n the presence of a base.

16. A method according to claim 14 or 15, wherein the nitrogen atoms of the Z' and Y' are protected by methanesulfonyl and/or 4-tolylsulfonyl and/or diethylphosphoryl.

17. A method according to claim 14, 15 or 16, wherein the deprotecting is carried out in a mixture of HBr and acetic acid in the case of methanesulfonyl and 4-tolylsulfonyl protection or by HCl in THF or dioxane in the case of diethylphosphoryl protection.

18. A pharmaceutical composition comprising as active ingredient a linked cyclic compound of general formula I,
Z - R - A - R' - Y (I)
in which Z and Y are independently cyclic polyamine moieties having from 9 to 32 ring members and from 3 to 8 amine nitrogens in the ring spaced by 2 or more carbon atoms from each other,
A is an aromatic or heteroaromatic moiety,
R and R' are each a substituted or unsubstituted alkylene chain or heteroatom containing chain,
or an acid addition salt or metal complex thereof, in admixture or association with a pharmaceutically acceptable diluent or carrier.
